# EUROPEAN PATENT APPLICATION

(11) **EP 3 607 959 A1**
(43) Date of publication of application: **12.02.2020**
(21) Application number: 18188413.1
(22) Date of filing: 10.08.2018
(51) Int. Cl.: A61K 36/67, A61K 36/9068, A61K 36/324, A61K 36/37, A61K 36/28, A61P 19/02, A61P 23/00, A61P 43/00

(54) **FORMULATIONS CONTAINING LIPOPHILIC EXTRACTS OF SPICY EDIBLE PLANTS USEFUL IN CONTROLLING PAIN AND INFLAMMATION**

(71) Applicant: Mehta, Raman, Kowloon (HK)
(72) Inventor: Mehta, Raman, Kowloon (HK)
(74) Representative: Zardi, Marco

(57) **Abstract**

The present invention concerns with new compositions containing lipophilic extracts of *Piper nigrum, Zingiber officinale, Boswellia serrata* and other plants used as adjuvant, prepared using as a solvent fluorinated hydrocarbons fluids in subcritical conditions. The compositions properly formulated are useful for topical and systemic treatment of pain and inflammation. The formulations are also active in muscle spasticity and neuropathic pains.

The formulations according to the invention, exerting a powerful synergistic effect, are particularly useful in the treatment of osteoarthrosis, osteoarthritis and arthritis psoriatic pain alone or in combination with specific drugs.

By topical application the formulation in form of creams, lotions or massage oils are indicated to reduce superficial and deep pain, edema and irritation of any origins.

## Description

The plants used in the present invention are edible plants used worldwide as a food and very often as medicinal items in ayurvedic system of medicine. Traditional medicine shows innumerable examples combinations of plants, often admixed in very large variety, used for a broad spectrum of different medical indications, yet with little knowledge of the full potential of each plant, and with variable results of the product due to the heterogeneity of plant sources, variability of extraction methods, different treatment methods, etc. Traditional medicine product, despite the apparent natural patient-friendly nature, involve complex interactions of active molecules, often non-fully explored as to tolerability, side effects, toxicity; they also expose the human body to a globally high amount of active principles, some of them possibly unnecessary for the specific condition to be treated. The use of non-ideal combinations translates into a low therapeutic efficacy, with the need to increase dosages and/or prolong the treatment over time, thus further exposing the organism to non-targeted treatments and potentially toxic drug amounts. Accordingly, modern medicine is since long active in rationalizing the use of these agents and components thereof, studying new medical indications, wanted and unwanted interactions, identifying possible synergisms, in the effort to provide the patient with most active combination of natural drugs, being closely tailored to his/her medical condition, with an eye to limiting patient's exposure to foreign molecules, both for in term of amounts and duration of treatment.

The plants on which the present invention Is based are basically three, herein always present in all combination and formulations, namely *Zingiber officinale, Piper nigrum, Boswellia serrata.*

*Zingiber officinale,* mainly used as spices in Far East and India entered also in the last decades in western countries for similar uses and in form of extracts in several nutraceutical products for treatment of nausea, gravidic vomit and in combination with other plants, for intestinal troubles. The ginger roots is one the most heavily consumed dietary substances in the world; ginger was first cultivated in Asia where it had been used as a medicinal plant for thousands of years before entering Western countries as a panacea. The active ingredients are considered gingerols, shoagols and gingerones. Ginger roots contain approximately 1.0 to 2.5 % of the gingerols, the pungent compounds which are responsible for anti-nausea, anti-inflammatory and analgesic effects. In ginger oil all of the pungent compounds contain the vanillyl (4-hydroxy-3-methoxyphenyl) moiety and a ketone functional group in their structure. Gingerols are the most abundant compounds in fresh roots and several gingerols of various chain length (n6 to n10) are present with the most abundant being 6-gingerol. The mechanism of action of gingerols and shoagols concerns the inhibition of inflammation mediators like NFkB, cannabinoid receptors CB2 and they are agonists of the vanilloid receptors RTPV1 involved in pain. Several *in vitro* tests and few *in vivo* are supporting this action in literature. In *in-vivo* studies the results are erratic mainly due to scarce bioavailability, rapid degradation and conjugation in the liver. The bioavailability of these molecules is the main problem and for these reasons suitable combinations with other compounds possibly endowed with synergistic effect in improvement and optimization of bioavailability are desired. Some problems are already partially addressed with some traditional combinations although without scientific justification and reproducibility. In the countries where the extracts are still used traditionally, they are combined with other ingredients. In western countries lipophilic extracts of *Zingiber officinale* are preliminarily used in oncology for anticipatory nausea and for treatment of inflammatory diseases even if the results are contradictory for the above reasons. In controlled clinical studies new formulations are necessary to confirm the properties of the extracts.

In controlled studies performed by the present inventors, within the present invention a surprising synergistic effect with Ginger oil was found for the *Piper nigrum* and *Boswellia serrata* lipophilic extracts, preferably obtained by Freon extraction according to the processes described in the present examples. The extraction can be carried out on the single plants alone or preferably on the prefixed combination of the starting vegetal material.

*Piper nigrum* or Black pepper is a spice commonly used in many parts of the world for flavor. Extracts of black pepper are largely used, through its active component piperine is known to modify metabolism in humans inhibiting the transport pump and interact with Cytochromes. A process in the liver namely glucuronidation, which conjugates several molecules changing their pharmacokinetics is inhibited by piperine.

Despite this apparently negative behavior of piperine, *Piper Nigrum* was found herein to improve the efficacy of other molecules according to the claimed combination. In black pepper extract, apart from the piperine content, there are other molecules, the isobutylamides, the most important being pellitorine, the strongest pungent molecule of the pepper lipophilic extract. Pellitorine interacts with vanilloid receptors RTPV1, saturating these receptors reduces specifically pain and inflammation. In the present invention is inter alia useful in increasing the absorption of other molecules of the present combination, for instance the gingerols which are glucuronidated and would be otherwise partially eliminated. The same action is useful in reducing the glucuronidation of other phenol groups present in active molecules of plant oils used in combination in the present invention. This is the case of some molecules present in *Boswellia serrata* oil, namely Serratol which is an agonist of Vanilloid receptor RTV3 and other mono and sesquiterpenes. The Vanilloid receptors RTV3 are involved in inflammation. In particular, Serratol is a very active anti-inflammatory substance and it would be desirable to obtain an extract of Boswellia Serrata containing this particular agent in high amounts. As experimentally supported by the annexed Examples, in an embodiment of the present invention, Boswellia serrata lipohilic extracts with high amounts of Serratol were unexpectedly obtained and most advantageously used in the present combinations. The other monoterpenes present in many plants in different concentration are natural vehicles of the active principles.

The extracts used in this invention are new lipophilic extracts prepared for the first time by extraction with Freon alone or in presence of suitable solvent as reported in the examples.

### SUMMARY OF THE INVENTION

It is now been discovered that a combination of extracts of specific plants used in Ayurvedic and Unani systems of medicine, preferably extracted with the new technological approaches described herein, are endowed with unpredictable, powerful effects in human treatment of inflammation and related pain. It is now been discovered that the combination of lipophilic extracts of Piper *nigrum, Zingiber officinale, Boswellia serrata,* particularly for topical administration, produces a potent, synergic analgesic and anti-inflammatory effect which is greater than can be obtained with uncombined extracts and even superior to several synthetic drugs. The combination according to the invention can be used in the treatment of pain of all kinds, particularly joint and muscle pain, arthrosis and arthritis, head ache and diabetes pains.

### DETAILED DESCRIPTION OF THE INVENTION

The term "lipophilic extract" is herein broadly referred to any extract obtained with a lipohilic solvent. The lipophilic solvent can be a liquid substance at ambient temperature and pressure; alternatively, it can be a non-liquid substance (typically a gas) at room temperature and pressure, which is brought to the liquid state at modified (subcritical) conditions of temperature and pressure, for extraction purpose. The lipophilic extract concentrates the water-insoluble or scarcely soluble ingredients of the plant, while containing a lower or nil amount of the hydrosoluble ones, depending on their degree of solubility into the lipophilic solvent. In the invention, lipophilic extracts of *Piper nigrum, Zingiber officinale, Boswellia serrata,* are preferably obtained by extraction with fluorinated hydrocarbons (e.g. Freon) in subcritical conditions (i.e. in the liquid state) without further purification with a great advantage in terms of economic balance and solvent quantity elimination.

The weight ratio among the three main components in the combination according to the present invention is about 1:1:2.5, where the term "about" indicates a variation of up to 20% for each of the above ratios. Particularly active resulted the combined extract extracted with Freon on the mixture of grinded biomasses in varying ratios which are regulated according to the analytical content of active principles of the biomasses, in particular: gingerols for *Zingiber officinale;* piperine and pellitorine for *Piper nigrum;* boswellic and acetylboswellic acids, incensol and in particular serratol for *Boswellia serrata.*

In a particularly preferred embodiment, the single extracts according to the invention are extracted with Freon alone or, preferably, in presence of suitable polar organic solvent (herein also referred as modifier); examples of suitable modifiers are methanol, ethanol, propanol, i-propanol, butanol, acetone, ethyl acetate, acetonitrile, dimethyl sulfoxide, dimethyl formamide; solvents like acetone or ethanol, allowed on regulatory point of view in food preparation, are especially preferred. The extraction procedure consists in putting the grinded material in individual cages in a suitable steel vessel under pressure with Freon circulation at a temperature between 25 and 45°C, preferably 35°C and pressure between 5 and 15/Kg/cm² preferably 8-9/Kg/cm² . The quantity of the circulating solvent, expressed as biomass/solvent ratio, is of about 1/6 for an extraction time of 3 to 10 hours, normally 5-6 hours for the completion of extraction. The solvent is evaporated in the evaporator where the extract is recovered.

In the case of the use of modifiers the process remains the same with the difference that, after a calculated time, the polar organic solvent is gradually added and the mixture is circulating until the completion of the extraction. The equipment is of course studied for perfect separation of solvent and extract recovery. The details will be reported in the examples.

The extracts are collected in the evaporator and after elimination of the water if present by mechanical separation can be directly diluted in oils in presence of surfactants or formulated in suitable excipients for human administration.

The invention includes a method of preparing the above described combination. In its general provision, the method comprising providing and compounding together lipophilic extracts of *Zingiber officinale, Piper nigrum and Boswellia serrata* ad described above. The above three extracts can be obtained as single raw products, or they can be prepared at once; if prepared at once, they can be prepared as separate extracts and then compounded together; alternatively, a single combined extract can be prepared by subjecting to extraction a mixture of the three plants; in this case, the above referred providing and compounding steps will be combined in one single step. The plants of the concerned three plants to be extracted, be it alone or in combination, are preferably comminuted or even pulverized, to ease the extraction process. Relevant plant parts useful for extraction purpose are: root and rhizome for *Zingiger officinale;* seed for *Piper Nigrum,* resin for *Bosewellia serrata.* As described above, any method of obtaining lipohilic extracts is contemplated. Preferably, extraction is performed with a fluorinated hydrocarbon (e.g. Freon) in subcritical conditions, optionally in presence of a polar solvent (modifier) as described above.

A further object of the present invention is a pharmaceutical composition comprising the combination of lipophilic extracts of *Zingiber officinale, Piper nigrum and Boswellia serrata,* described above.

In the present composition, the above combination can represent the sole active agents present, or it may be in presence of a limited amount of other active agents, also typically in form of extracts. When in presence of other active agents, the combination of *Zingiber officinale, Piper nigrum and Boswellia serrata,* will represent at least 50% by weight, preferably at least 75%, more preferably at least 90% of the overall amount of active agents present in the composition.

The other active agents, when present, are preferably chosen among extracts of plants being known as additional sources of the present molecules of interest. For example, pellitorine is further present in *Anacyclus phyrethrum*; another molecule of interest, celastrol, is present in *Celastrus paniculatus*. Celastrol a triterpenoid isolated from traditional Chinese medicine, is a promising drug for the treatment of various inflammatory and autoimmune diseases. Reverse transcription polymerase chain reaction, western blotting and ELISAs were performed to examine the effect of celastrol on interleukin (IL) 1β induced inflammation. The results demonstrated that celastrol significantly attenuated the expression of IL 6, IL 8, cyclooxygenase (COX) 2 and intercellular adhesion mol. 1 (ICAM 1), and inhibited IL 1β induced increases in the expression of IL 6, IL 8, ICAM 1 and COX 2. Further investigation revealed that celastrol suppressed the IL 1β-induced inflammatory responses through inhibiting the activation of nuclear factor (NF). Extracts of both *Anacyclus phyrethrum* and *Celastrus paniculatus* are advantageously present in the compositions, particularly in massage oils for peripheral pains.

In addition to the above active mentioned active agents, the pharmaceutical compositions will contain customary pharmacologically non-active ingredients. These will be selected, in quality and quantity, depending on the specific chosen administration form, and chemical-physical nature of the extracts to be formulated, etc. Among the non-active ingredients, there can be mentioned: liquid, solid or semisolid carriers, surfactants, wetting agents, buffers, tonicity adjusting agents, fluidifying agents, thickeners, disintegrants, preservatives, dyes, flavors, etc.

The present combinations and compositions can be formulated and supplied in any known dosage form, depending on the desired administration route and patient conditions: a non-limitative list of dosage forms comprises solutions, dispersions, emulsion, powders, creams, ointments, patches, plasters, films, gels, tablets, troches, capsules, syrups, aerosols, suppositories, infusions, and the like.

The present combinations and composition can be administered by any known administration route, including oral, buccal, topical, transdermal, inhalatory, intramuscular, intravenous, etc. Particularly preferred is the oral or topical administration.

For oral administration the formulation contains the extracts of *Zingiber officinale, Piper nigrum and Boswellia serrata,* preferably in presence of phospholipids and omega 3 fatty acids or extracts containing them.

For topical administration according to one embodiment of the invention, an oily formulation for massage typically contain between 1.5-4.5 % of ginger oil, preferably 3, between 5-15% of *Piper nigrum* extract preferably 9%, between 2-7% of *Boswellia serrata* oily extract, preferably 5%, between 0-3% of *Celastrus paniculatus,* preferably 1%. *Lavandula* oil can be added to improve the pleasure and efficacy of the combination. The extracts in massage oil are incorporated in apricot oil in presence of 2% of phospholipids. The same extracts incorporated in lotions, creams and ointments can have different percentage according to the pathology or discomfort to be treated.

A further object of the present invention is represented by the above described combinations and pharmaceutical compositions, for use in therapy. In particular they are used in the treatment or prevention of inflammatory diseases; examples of inflammatory diseases are peripheral pain, inflammatory status of different origin, pain associated with muscle spam like stiffness, stiff neck, painful lumbago, osteoarthrosis, osteoarthritis, psoriatic arthritis and gout. The composition according to the invention can also be used in cosmetic field to reduce inflammation and irritative states and itching. The treatment can be performed one to four times a day applying the formulation to the part of the body affected by the painful pathology. By oral administration the extracts can be incorporated e.g. in tablets or hard capsules suitable for oils or preferentially in soft gelatin capsules.

Useful oral dosage units for the combination of the invention will contain, subject to the above described mutual weight ratios): 20-60 mg of *Zingiber officinale;* 20-60 mg of *Piper nigrum;* 80-120 mg of *Boswellia serrata*; a preferred oral composition will contain 40 mg of *Zingiber officinale*; 40 mg of *Piper nigrum*; 100 mg of *Boswellia serrata*.

The following non-limitative examples, illustrate the invention in detail.

### EXPERIMENTALS

### Example 1 Preparation of Zingiber officinale extract using extracting solvent (Freon Hydrofluorocarbon R134A) and modifier solvent (acetone).

50 Kg of grinded roots and rhyzomes of *Zingiber officinale* having a content in gingerols and Shoagols of 1.2% are located in the steel extractor; vacuum is created in the vessel, evaporator and connecting lines and after air elimination Freon (HFC Hydrofluorocarbon R134A) is introduced (250 Kg) and the solvent circulation is then started at a pressure of 8-9 Kg/cm² (35°C) for 35 min while circulation of Freon is continued with acetone (200 L) is dosed at 1-1,5 lit/ minute for 3 hours.

Afterwards, Freon is recovered in the evaporator and then transferred to the storage container. Extracted material is taken out of evaporator and after filtration through cloth to remove traces of plant material the acetone is distilled off completely under vacuum at 40°C to isolate the oily mass. 2.2 Kg of *Zingiber officinale oil* has been obtained having the following characteristics: Light brown mobile liquid, spicy characteristic; Density 20°C 0.97 Gingerols and Shoagols not less then 25%

### Example 2 Preparation of Piper nigrum seeds extract using extracting solvent (Freon) and modifier solvent (ethanol).

50 Kg of grinded seeds of *Piper nigrum* are located in the steel extractor for treatment of biomass under pressure with Rreon; vacuum is created in the vessel, evaporator and connecting lines and after air elimination Freon is introduced (250 Kg) and the solvent circulation is then started at a pressure of 8-9 Kg/cm² (35°C) for 35 min while circulation of Freon is continued with ethanol (200 L) is dosed at 1-1,5 lit/ minute for 3 hours.

Afterwards, Freon is recovered in the evaporator and then transferred to the storage container. Extracted material is taken out of evaporator and after filtration through cloth to remove traces of plant material the acetone is distilled off completely under vacuum at 40°C to isolate the oily mass. 1.43.Kg of *Piper nigrum oil* has been obtained having the following characteristics: Brown mobile oil with strong spicy characteristic; Density 20°C 0.90. Piperine content by HPLC 38.6% Pellitorine 16%; several additional picks as in fingerprint reported.

### Example 3 Preparation of Boswellia serrata resin extract using extracting solvent (Freon).

50 Kg of grinded resins of *Boswellia serrata* having a content in total boswellic acids of 26% are located in the steel extractor for treatment of biomass under pressure with freon; vacuum is created in the vessel, evaporator and connecting lines and after air elimination Freon is introduced (250 Kg) and the solvent circulation is then started at a pressure of 8-9 Kg/cm² (35°C) for 5 hours for completion of extraction.

Afterwards, Freon is recovered in the evaporator and then transferred to the storage container. Extracted material is taken out of evaporator and after filtration through cloth to remove traces of plant material 8.9.Kg of *Boswellia serrata oil* has been obtained having the following characteristics: Pale Yellow mobile liquid, characteristic woody, spicy camphorous odor; Density 20°C 0.850; GC MS (Content in Boswellic and 3-acetylboswellic acid 0.23 and 0.2% and serratol 3%)

### Example 3 bis Preparation of Boswellia serrata resin extract using extracting solvent (Freon) and modifier solvent (acetone)

50 Kg of grinded resin of Boswellia serrata having a content in total boswellic acids of 26%. are located in the steel extractor; vacuum is created in the vessel, evaporator and connecting lines and after air elimination Freon, HFC (Hydrofluorocarbon R134A) is introduced (250 Kg) and the solvent circulation is then started at a pressure of 8-9 Kg/cm² (35°C) for 35 min while circulation of Freon is continued with acetone (200 L) is dosed at 1-1,5 lit/ minute for 3 hours.

Afterwards, Freon is recovered in the evaporator and then transferred to the storage container. Extracted material is taken out of evaporator and after filtration through cloth to remove traces of plant material the acetone is distilled off completely under vacuum at 40°C to isolate the oily mass.12.5 Kg of *Boswellia serrata oil* has been obtained having the following characteristics: 1.5 and 1.7 % of boswellic and 3-acetylboswellic acids and 25% in serratol. Significantly, the use of modifier solvent has strongly increased the amount and concentration of recovered serratol.

### Example 4 Preparation of Celastrus paniculatus extract using extraction solvent (Freon)

50 Kg of grinded seeds of *Celastrus paniculatus* are located in the steel extractor for treatment of biomass under pressure with freon; vacuum is created in the vessel, evaporator and connecting lines and after air elimination Freon is introduced (250 Kg) and the solvent circulation is then started at a pressure of 8-9 Kg/cm² (35°C) for 5 hours for completion of extraction.

Afterwards, Freon is recovered in the evaporator and then transferred to the storage container. Extracted material is taken out of evaporator and after filtration through cloth to remove traces of plant material.

### Example 5 Preparation of new oily extract combining different plant biomasess in the extraction process.

50 Kg of a mixture of grinded resins of *Boswellia serrata, Piper migrum unriped seeds, Zingiber officinale roots and rhyzomes* in ratio respectively by weight of 10:4:4 after accurate homogenization are located in the steel extractor for treatment of biomass under pressure with freon; vacuum is created in the vessel, evaporator and connecting lines and after air elimination Freon (Hydrofluorocarbon R134A) is introduced (250 Kg) and the solvent circulation is then started at a pressure of 8-9 Kg/cm² (35°C) for 1 hour while circulation of Freon is continued with ethanol (200 L) is dosed at 1-1,5 lit/ minute for 3 hours for completion of extraction. Afterwards, Freon is recovered in the evaporator and then transferred to the storage container. Extracted material is taken out of evaporator and after filtration through cloth to remove traces of plant material the ethanol is distilled off completely under vacuum at 40°C to isolate the oily mass.

### Example 6 Preparation of soft gelatin capsules containing Boswellia serrata, Piper nigrum and Zingiber officinale oils prepared according to examples 1, 2 3.

Each soft gelatin capsule contains:

| | |
|---|---|
| *Boswellia serrata oil* | 40 mg |
| *Piper* nigrum oil | 20 mg |
| *Zingiber officinale* oil | 40 mg |
| Lecithin liquid | 50 mg |
| Flaxseed oil | 120 mg |

### Example 7 Preparation of massage oil

### Blending procedure for preparation of Standardized Phytoextract of Boswellia serrata, Zingiber officinale, Piper nigrum, Celastrus paniculatus and Lavandula officinalis oils

Step 1 Sunflower lecithin (1,5 g) is added to Apricot oil (17,5 ml) and the mixture is heated at 55-60°C with stirring to form an homogeneous mixture. The mixture is then cooled to 25°C and used as such.
Step 2 In an other vessel containing apricot oil (11,5 ml) at room temperature (25°C) under stirring are added 1 ml of ginger oil (Example 1), *Celastrus oil* (Example 4) 0.4 ml and *Piper nigrum* oil (Example 2) 3 ml and the resulting solution is stirred until formation of homogeneous mixture,
Step 3 Mixture of step 1 is added to mixture from step 2 under stirring along with 2 ml of *Boswellia serrata* oil and 0.3 ml of *Lavandula officinalis* oil.

The mixture is stirred at room temperature until homogeneous mixture of blend is formed

### Example 8 Evaluation of the analgesic activity in rats of single extracts and combination according to the example 6 - assessment of synergism

The analgesic activity of the composition was evaluated with tail-flic test in rats. According to the literature before treatment 3 determinations were conducted to ensure the validity of the experiment. The parameters used were 15V of radiant heat and a 15 second cut-off wit evaluation of the tail-flick. The animals were treated with 0.2 ml of the composition of example 6 at 4 cm from the start of the tail or with sunflower oil. The analgesic effect was measured after 15 and 30n minutes after topical application. The single extracts are diluted in sunflower oil in the concentration present in the formulation of the example 6. The results are reported in the table 1.

**Table 1**

| **TREATMENT** | **LATENCY TIME** | | | |
|---|---|---|---|---|
| | **after 15 min** | **% increase** | **after 30 min** | **% increase** |
| | | | | |
| Carrier Sunflower oil | 4.8 ± 0.21 | | 4.4 ± 0.32 | |
| FormulationEx.6 | 13.9±0.51 | 191 | 12±0.44 | 86.2 |
| Zingiber officinale | 6.4±0.54 | 36.2 | 4.6±0.49 | 29.4 |
| Piper nigrum | 6.1±0.41 | 32.6 | 5.2±0.38 | 10.4 |
| Boswellia serrata | 4.7±0.18 | | 4.6±0.38 | |

The formulation of Example 6 clearly provides an effect being clearly superior to the sum of effects obtained by the single extracts. This shows the presence of a synergistic effect for the composition of the invention.

### Example 9 Evaluation of the analgesic activity in patient of extracts combination according to the example 6 (capsules)

Soft gelatin capsules prepared according to the example 6 containing 40 mg of Ginger oil of Example 1, 40 mg of *Piper nigrum* extract according to the example 2 and 100 mg of *Boswellia serrata oil* according to the example 3 (AB) were given to 25 patients suffering from intense joint pain, reduced mobility and quality of the life. The efficacy of the new formulation has been tested against placebo to ascertain its pharmaco-dynamic action. Patient controls have been carried out after 4 and 8 weeks. The clinical results are reported in table 2.

**Table 2**

| | **Example 6** | | | | | **Controls** | | | | |
|---|---|---|---|---|---|---|---|---|---|---|
| | **Pain** | **Mobility** | **RM** | **PWD** | **PFR** | **Pain** | **Mobility** | **RM** | **PWD** | **PFR** |
| | | | | | | | | | | |
| inclusion | 6.8;1.1 | 3.2;0.6 | 6 | 88;12 | 388;23 | 6.5;0.5 | 3.3;0.2 | 5 | 91;11 | 382:22 |
| 4 wks | 2.6;1* | 6.3;1* | 3* | | | 5.4;0.2 | 3.1;0.4 | 4 | | |
| 8 wks | 2.1;0.3* | 7.1;0.3* | 1* | | | 5;0.7 | 2.6;0.3 | 4 | | |
| 12 wks | 1.3;0.4* | 7.8;0.4* | 0* | 211;18* | 335;12* | 4.7;0.6 | 3;0.3 | 3 | 166;12 | 374;18 |

| | | | | | | | | | | |
|---|---|---|---|---|---|---|---|---|---|---|
| *RM= Rescue medication; PWD= painfree walk distance; PFR= plasma free radicals (Carr Units)* | | | | | | | | | | |

From this table it is clear the analgesic activity of the combination; no tolerability problems have been noticed.

### Example 10 Evaluation of the analgesic activity in (peripheral pain) patient of extracts combination according to the example 7 (massage oil)

As far as other formulations is concerned for pain treatment a massage oil has been realized of particular interest in neck pain or joint treatment. The oil could be administered also through iontophoresis or incorporated in plasters.

A formulation for topical massage as reported in example 7 has been tested in different groups of patients suffering from peripheral pains. A group of 22 subjects suffering from neck pain was treated by physiotherapist three times a day for one week evaluating the pain reduction and the time necessary for an acceptable time reduction.

The results are reported in table 3 along with a comparison with standard therapy (control).

**TABLE 3: MAIN SYMPTOMS. VISUAL ANALOGUE SCALE LINE SCORE.**

| **Symptom** | **Patient group** | **T=0 (inclusion)** | **T= 1 week** | **T = 2 weeks** |
|---|---|---|---|---|
| | | | | |
| *Spontaneous Pain* | Ex. 7 | 2.33;0.3 | 1.24;0.2 | *0.3;0.1 |
| | Control | 2.2;0.4 | 1.6;0.2 | 0.7;0.2 |
| | | | | |
| *Pain on motion* | Ex. 7 | 2.6;0.4 | 1.33;0.1 | *0.8;0.2 |
| | Control | 2.6;0.32 | 1.8;0.2 | 1.3;0.2 |
| | | | | |
| *Stiffness* | Ex. 7 | 2.33;0.2 | 1.2;0.1 | 0.3;0.1 |
| | Control | 2.42;0.1 | 1.84;0.3 | 1.6;0.3 |
| | | | | |
| *Altered mobility* | Ex. 7 | 2.37;0.1 | 1.1;0.1 | 0.2;0.1 |
| | Control | 3.4;0.3 | 2,1;0.2 | 1.8;0.2 |
| | | | | |
| *Altered working capacity* | Ex. 7 | 2.22;0.3 | 0.62;0.2 | 0.3;0.2 |
| | Control | 2.2;0.2 | 2.1;0.2 | 0.6;0.2 |

All measurements reached statistical significance (p<0.05). The data in table 3 show, for the composition of the invention, consistently high performance in terms of pain/stiffness reduction and restored mobility; further, the recourse to rescue medication was reduced, and tissue microcirculation (tested with laser Doppler) increased; iontophoresis on the composition of example 7 obtained a skin penetration of the oil components of 4.9 minutes.

### Example 11 Evaluation of the analgesic activity in (osteoarthritis) patients of extracts combination according to the example 6 (capsules); comparison with ketoprofene

In a controlled clinical trial a group of patients suffering from osteoarthritis was treated in a double blind study with the combination according to example 6 or with ketoprofene as comparative drug substance. 64 patients divided in two homogeneous groups as reported in Table 4 were treated twice a day for 6 months with combination 6 or ketoprofene evaluating the relative efficacy and tolerability.

**Table 4 Baseline characteristic of 64 patients with knee osteoarthritis and clinical results**

| **Baseline characteristics** | **Ketoprofene (n=30)** | **Example 6 (n=34)** | **P-value** |
|---|---|---|---|
| | | | |
| Mean age ±SD (years) | 60.9±6.1 | 60.2±6.8 | 0.456 |
| Mean BMI ±SD (Kg/m²) | 26.4±3.1 | 26.2±3.6 | 0.92 |
| Mean duration of pain | 52.0±53.1 | 51.0±52.2 | 0.89 |
| Routinely taking pain | 26 | 28 | 0.22 |
| WOMAC total±SD | 5.2±1.51 | 5.3±1.81 | 0.61 |
| WOMAC pain±SD | 5.4±1.52 | 5.3±1.81 | 0.87 |
| WOMAC stiffness±SD | 5.2±2.58 | 5.1±2.65 | 0.85 |
| WOMAC functions±SD | 5.1±1.96 | 5.3±2.01 | 0.46 |
| Walk distance 6 min | 304±81.96 | 310±82.01 | 0.482 |

The clinical results are proving the efficacy of this new combinations of natural products.

## Claims

1. A combination consisting of lipophilic extracts of *Piper nigrum, Zingiber officinale* and *Boswellia serrata.*

2. The combination of claim 1, wherein said extract of *Zingiber officinale* contains at least 10% by weight gingerols.

3. The combination of claims 1-2, wherein said extract of *Piper nigrum* contains at least 10% by weight piperine and at least 1% by weight pellitorine.

4. The combination of claims 1-3, wherein said extract of *Boswellia serrata* contains at least 0.1 % by weight boswellic acid, at least 0.1 % by weight of acetylboswellic acid and at least 2% serratol.

5. The combination of claim 1-4, wherein said extract of *Boswellia serrata* contains at least 20% by weight serratol.

6. The combination of claims 1-5, wherein one, two or all of said extracts are obtained by extraction of the corresponding plants or mixtures thereof with a fluorinated hydrocarbon fluid in subcritical conditions, optionally in presence of a polar organic solvent.

7. The combination of claims 1-6, wherein the fluorinated hydrocarbon fluid is Freon and the polar organic solvent is selected from: methanol, ethanol, propanol, i-propanol, butanol, acetone, ethyl acetate, acetonitrile, dimethyl sulfoxide, dimethyl formamide and mixtures thereof.

8. The combination of claims 1, wherein the lipophilic extracts lipophilic extracts of *Piper nigrum, Zingiber officinale* and *Boswellia serrata* are present in the respective weight ratios of about 1:1:2.5.

9. A method to prepare a combination according to claims 1-9, comprising providing extracts of *Piper nigrum, Zingiber officinale* and *Boswellia serrata* and compounding them together to form the combination.

10. Pharmaceutical composition comprising the combination of lipophilic extracts described in claims 1-8.

11. The composition of claim 10, further comprising extracts of one or more among: *Celastrus paniculatus, Anacyclus pyrethrum, Echinacea sp.*

12. The composition of claims 10-11, wherein the extracts of *Piper nigrum, Zingiber officinale* and *Boswellia serrata* represent, as a whole, at least 75% by weight of the active principles present in the composition.

13. The combination or composition of claims 1-8, 10-12 for use in therapy.

14. The combination or composition of 13, for use in treating and/or preventing inflammatory disorders.

15. The combination or composition of 14, for use in treating and/or preventing an inflammatory disorder selected from: peripheral pain, inflammatory status of different origin, pain associated with muscle spam like stiffness, stiff neck, painful lumbago, joint pain, muscle pain, osteoarthrosis, osteoarthritis, psoriatic arthritis, headache, diabetic pain, gout.
